# EUROPEAN PATENT SPECIFICATION

(11) **EP 4 047 007 B1**
(45) Date of publication and mention of the grant of the patent: **05.06.2024**
(21) Application number: 19949541.7
(22) Date of filing: 18.10.2019
(51) Int. Cl.: C07K 7/06, C07K 7/08, A61K 38/08, A61K 38/10, A61P 19/02, A61P 25/02, A61P 25/04, A61P 25/06, A61P 25/00, A61P 29/00

(54) **PEPTIDE AND USE THEREOF IN PREPARATION OF DRUG FOR TREATING INFLAMMATORY DISEASES AND PAIN**
PEPTID UND SEINE VERWENDUNG ZUR HERSTELLUNG EINES ARZNEIMITTELS ZUR BEHANDLUNG VON ENTZÜNDLICHEN ERKRANKUNGEN UND SCHMERZEN
PEPTIDE ET SON UTILISATION DANS LA PRÉPARATION D'UN MÉDICAMENT POUR LE TRAITEMENT DE MALADIES INFLAMMATOIRES ET DE LA DOULEUR

(43) Date of publication of application: 24.08.2022
(73) Proprietor: Pell Bio-Med Technology Co., Ltd., Taipei City (TW)
(72) Inventor: LIN, Chen-Lung, Kaohsiung City, Taiwan 82151 (TW)
(74) Representative: Becker, Eberhard
(86) International application number: PCT/CN2019/111788
(87) International publication number: WO 2021/072724

(56) References cited:
- EP-A2- 0 341 209
- WO-A1-2013/173941
- WO-A1-2016/165101
- WO-A1-2019/103203
- WO-A1-2020/024988
- CN-A- 104 321 337
- CN-A- 104 321 337
- CN-A- 107 847 550
- CN-A- 107 849 091
- CN-A- 107 849 091
- ZHENG YUQIU ET AL: "Neurotropin inhibits neuroinflammation via suppressing NF-kB and MAPKs signaling pathways in lipopolysaccharide-stimulated BV2 cells", JOURNAL OF PHARMACOLOGICAL SCIENCES, vol. 136, 27 February 2018 (2018-02-27), pages 242-248, XP055802405, DOI: 10.1016/j.jphs.2018.02.004
- AKIO SAITO ET AL: "Cloning and Sequencing of cDNA Coding for Rabbit ƒ¿-1-Antiproteinase F: Amino Acid Sequence Comparison of alpha-1-Antiproteinases of Six Mammals", JOURNAL OF BIOCHEMISTRY, vol. 109, no. 1, 1991, pages 158-162, XP055179611,
- BECK A ET AL: "Stability and CTL activity of N-terminal glutamic acid containing peptides", JOURNAL OF PEPTIDE RESEARCH, BLACKWELL PUBLISHING LTD, OXFORD; GB, vol. 57, no. 6, 1 June 2001 (2001-06-01), pages 528-538, XP002303302, ISSN: 1397-002X, DOI: 10.1034/J.1399-3011.2001.00895.X
- LAWRENCE W DICK ET AL: "Determination of the origin of the N-terminal pyro-glutamate variation in monoclonal antibodies using model peptides", BIOTECHNOLOGY AND BIOENGINEERING, JOHN WILEY, HOBOKEN, USA, vol. 97, no. 3, 10 November 2006 (2006-11-10), pages 544-553, XP071033141, ISSN: 0006-3592, DOI: 10.1002/BIT.21260
- Zheng Yuqiu, Wenli Fang, Shengnuo Fan, Wang Liao, Ying Xiong, Shaowei Liao, Yi Li, Songhua Xiao, Jun Liu: "Neurotropin inhibits neuroinflammation via suppressing NF-kB and MAPKs signaling pathways in lipopolysaccharide-stimulated BV2 cells", Journal of Pharmacological Sciences, vol. 136, 27 February 2018 (2018-02-27), pages 242-248, XP055802405, DOI: 10.1016/j.jphs.2018.02.004
- Miura T, Okazaki R, Yoshida H, Namba H, Okai H, Kawamura M.: "Mechanisms of analgesic action of Neurotropin on chronic pain in adjuvant-induced arthritic rat: Roles of descending noradrenergic and serotonergic systems", Journal of Pharmacological Sciences, Japanese Pharmacological society , Tokyo, JP, vol. 97, no. 3, 1 March 2005 (2005-03-01), pages 429-436, XP002726216, JP ISSN: 1347-8613, DOI: 10.1254/jphs.FPJ04050X
- Zhiqiang Wang, Zhang Yao, Hong Xuechuan, Xu Ping: "N-terminomics: proteomic strategies for protein N-terminal profiling", Chinese Journal of Biotechnology, Zhongguo Kexueyuan Weishengwu Yanjiusuo, Chinese Academy of Sciences, Institute of Microbiology, CN, vol. 32, no. 8, 25 August 2016 (2016-08-25), pages 1001-1009, XP055684259, CN ISSN: 1000-3061, DOI: 10.13345/j.cjb.150441

## Description

The present invention relates to a peptide, which can be used for treating an inflammatory disease and pain, and to the use thereof in manufacturing a medicament for treating an inflammatory disease.

Inflammatory responses, including redness, swelling, heat and pain, refer to a series of physiological responses of body tissue to stimuli such as injuries or pathogenic infections. Inflammatory response is one of the reactions that an organism reacts to exogenous pathogen and is one of the immune responses beneficial for the organism. However, inflammation lasting for a long period of time, namely chronic inflammation, may cause several inflammatory diseases, such as pollinosis, periodontitis, rheumatoid arthritis, etc. Cytokines have regulatory influence on inflammation. Several cytokines play a role in the inflammatory response. Tumor necrosis factor (TNF) is a multifunctional cytokine, which promotes the proliferation of macrophages or monocytes during inflammation response and activates the expression of cytokines in the downstream inflammatory response; therefore, it is a pro-inflammatory cytokine. Among all cytokines, tumor necrosis factor-α (TNF-α) is commonly used as an indicator for accessing inflammation. Besides, interleukin-6 also acts as a pro-inflammatory substance, which brings many monocytes and macrophages to the affected area and causes inflammatory responses.

The International Association for the Study of Pain (IASP) defines the pain as: an unpleasant sensory and emotional experience associated with actual or potential tissue damage. Pain is the unpleasant sensation of the nervous system. The common types of pain include: nociceptive pain, neuropathic pain, sympathetic pain, psychogenic pain, etc. In addition, pain can also be distinguished by body parts, time, such as acute or chronic, pathogenesis, and physiological characteristics. Among all pain, acute pain lasts for a shorter time period and is usually the alarm signal for tissue injury. Therefore, this type of pain will disappear after tissue recovery. Chronic pain lasts for a longer time period and usually is not the signal of tissue injury
but the signal for diseases. Patients with chronic pain are likely to be depressing, autistic, or irritable, requiring higher social costs. Thus, pain management has always been a significant medical issue.

WO2013/173941 A1 discloses a pain relieving peptide. WO 2020/024988 A1 and WO 2016/165101 A1 disclose small peptides with medical effect. Zheng et al. (Journal of Pharmacological Sciences, 136(4), 242-248) teach that Neurotropin (NTP) is a non-protein bioactive agent extracted from inflamed rabbit skin inoculated with vaccinia virus, is widely used for the treatment of chronic pain condition. Saito and Sinohara (The Journal of Biochemistry, 109(1), 158-162) teach the amino acid sequence of rabbit α-1-antiproteinase F. In addition, Beck et al. (The Journal of Peptide Research, 57(6), 528-538) and Dick et al. (Biotechnology and bioengineering, 97(3), 544-553) teach some examples of Glp-comprising peptide.

In light of the fact that there is an urgent need to develop novel anti-inflammatory painkillers, the present invention provides a peptide, which can be used for treating an inflammatory disease and pain. Further, the present invention provides use of the peptide for manufacturing a medicament for treating an inflammatory disease, wherein the peptide consists of an amino acid sequence of SEQ ID NO: 1, and the medicament comprises an effective amount of the peptide and a pharmaceutically acceptable carrier. Besides, the peptide can be used for treating pain. Therefore, the present invention also provides use of a peptide for manufacturing a medicament for treating an inflammatory disease, wherein the peptide consists of an amino acid sequence of SEQ ID NO: 1, and the medicament comprises an effective amount of the peptide and a pharmaceutically acceptable carrier.

The present invention further provides a peptide for treating an inflammatory disease, wherein the peptide consists of an amino acid sequence of SEQ ID NO: 1.

The present invention further provides a peptide for treating pain, wherein the peptide consists of an amino acid sequence of SEQ ID NO: 1.

The present invention further provides a method for treating an inflammatory disease, comprising administering to a subject in need thereof a therapeutically effective amount of a peptide, wherein the peptide consists of an amino acid sequence of SEQ ID NO: 1.

The present invention further provides a method for treating pain, comprising administering to a subject in need thereof a therapeutically effective amount of a peptide, wherein the peptide consists of an amino acid sequence of SEQ ID NO: 1.

Preferably, the peptide is consisting of an amino acid sequence of SEQ ID NO: 1. Preferably, according to the present invention, the "pharmaceutically acceptable carrier" includes, but is not limited to, solvents, emulsifiers, suspending agents, disintegrators, binding agents, excipients, stabilizing agents, diluents, gelling agents, preservatives, lubricants, surfactants and other similar carriers or the carriers that are suitable for the present invention.

Preferably, the aforementioned inflammatory disease includes ankylosing spondylitis, osteoarthritis, rheumatic arthritis, rheumatoid arthritis, traumatic arthritis, pyogenic arthritis, gouty arthritis, tuberculous arthritis, neuropathic arthritis, and hemophilic arthritis.

Preferably, the aforementioned pain includes neuropathic pain, inflammatory pain, musculoskeletal pain, postoperative pain, cancer pain, acute pain, and chronic pain.

Preferably, the aforementioned inflammatory pain includes ankylosing spondylitis pain, osteoarthritis pain, rheumatic arthritis pain, rheumatoid arthritis pain, traumatic arthritis pain, pyogenic arthritis pain, gouty arthritis pain, tuberculous arthritis pain, neuropathic arthritis pain, and hemophilic arthritis pain.

The aforementioned medicament may be an enteral or parenteral dosage form. The enteral dosage form includes, but is not limited to, enteric coated tablets, multilayer tablets, sugar-coated tablets, sublingual tablets, chewable tablets, troches, capsules, powder, syrups, solutions, emulsions, suspensions, mucilages, magmas, fluid extracts, extracts, spirits, elixirs, and tinctures, etc. of oral dosage form and enema. The parenteral dosage form includes, but is not limited to, dosage form for injection, ointments, lotions, liniments, and aerosols.

Preferably, the effective amount ranges from 0.0008 µg/kg to 815 µg/kg.

Preferably, the peptide of the present application is administered at a frequency of once per month to once per day. More preferably, the peptide of the present application is administered at a frequency of twice per month to twice per week. More preferably, the peptide of the present application is administered at a frequency of once per week.

More preferably, the peptide of the present application is administered with the aforementioned effective amount once per week.

According to the present invention, the effective amount is calculated based on the concentration of peptide SEQ ID NO: 1 ranging from 0.01 µg/kg to 10 mg/kg in the embodiments of the present description and in accordance with the guidance document "Estimating the Maximum Safe Starting Dose in Initial Clinical Trials for Therapeutics in Adult Healthy Volunteers" published by the U.S. Food and Drug Administration. For an adult human having body weight (b.w.) of 60 kg, the dose can be converted by the criteria: 12.3 × human recommended dose per kg b.w. per day = 1 × mouse dose.

More preferably, the effective amount ranges from 0.008 µg/kg to 410 µg/kg.

More preferably, the effective amount ranges from 0.008 µg/kg to 0.25 µg/kg.

More preferably, the effective amount ranges from 0.025 µg/kg to 0.25 µg/kg.

More preferably, the effective amount ranges from 80 µg/kg to 410 µg/kg.

More preferably, the effective amount ranges from 80 µg/kg to 250 µg/kg.

Preferably, the enteral dosage form is an oral dosage form.

Preferably, the parenteral dosage form is a dosage form for injection.

According to the present invention, "treating" refers to administering the peptide of the present invention to a patient in order to achieve the effect of treatment, wherein the effect of treatment refers to curing, mitigating, ameliorating or relieving a disease or related symptoms. According to the present invention, treating an inflammatory disease refers to ameliorating the inflammation-related symptoms, which is evaluated by the reduction of inflammation indicators in some embodiments. According to the present invention, treating pain refers to ameliorating the pain-related symptoms, which is evaluated by the increased threshold of pain perception in some embodiments; or by the increased paw withdrawal threshold (PWT) in some embodiments.

According to the present invention, the term "effective amount" refers to a dosage for the desired biological reaction, namely, a dosage for the desired treatment effect.

### IN THE DRAWINGS:

FIG. 1 shows the serum concentration of TNF-α in mice after administrated with 3 mg/kg the peptide of SEQ ID NO: 1 every week (experimental group) from the 4^{th} week to the 12^{th} week compared with the control group.
FIG. 2 shows the serum concentration of IL-6 in mice after administrated with 3 mg/kg the peptide of SEQ ID NO: 1 every week (experimental group) from the 4^{th} week to the 12^{th} week compared with the control group.
FIG. 3 shows the change in PWT of ipsilateral hind paw of mice over time after administered with 3 mg/kg the peptide of SEQ ID NO: 1 (experimental group) compared with the morphine group (10 mg/kg).
FIG. 4 shows the change in PWT of contralateral paw of mice over time after administered with 3 mg/kg the peptide of SEQ ID NO: 1 (experimental group) compared with the morphine group (10 mg/kg).
FIG. 5 shows the PWT of ipsilateral hind paw of mice after administrated with 1 mg/kg and 3 mg/kg the peptide of SEQ ID NO: 1 every week from the 4^{th} week to the 12^{th} week compared with the control group.
FIG. 6 shows the PWT of contralateral hind paw of mice after administrated with 1 mg/mL and 3 mg/mL peptide of SEQ ID NO: 1 every week from the 4^{th} week to the 12^{th} week compared with the control group.
FIG. 7 shows the PWT of the ipsilateral hind paw of mice after administered with 0.3 µg/kg, 1 µg/kg, 3 µg/kg the peptide of SEQ ID NO: 1 respectively, compared with the control group.
FIG. 8 shows the PWT of the contralateral paw of mice after administered with 0.3 µg/kg, 1 µg/kg, 3 µg/kg the peptide of SEQ ID NO: 1 respectively, compared with the control group.
FIG. 9 shows the antinociceptive activity of the peptide of SEQ ID NO: 1 in the acetic acid-induced writhing experiment and the maximum possible effect of the peptide of SEQ ID NO: 1 in the tail-flick test.

The technical means adopted for achieving predetermined invention purpose of the present invention is further explained through the accompanied drawings, following preparation examples and examples of the present invention.

### Preparation Example 1

The standard Fmoc strategy and microwave peptide synthesizer were applied to synthesize the peptide of Glp Glu Thr Ala Val Ser Ser His Glu Gln Asp used in the present invention, wherein the Glp is pyroglutamic acid (pyrrolidone carboxylic acid) (Cas Number 98-79-3). In general, Wang resin (0.6 mmol/g load) preloaded with D residues was weighed into a reaction vessel and added with fresh DMF (10-15 mL) before the synthesis and was ready to be swollen. The swelling time was set as 3 minutes (min) on the microwave peptide synthesizer.

The first and subsequent Fmoc groups were removed with 5 mL 20% Piperidine in DMF, served as the solution for "DEP" step, by a standard de-protection process (the first stage: a temperature of 75°C, power of 155 W, and a temperature holding time of 15 seconds; the second stage: a temperature of 90°C, power of 30 W, and a temperature holding time of 50 seconds), in order to free the N-terminal amine group.

Then, 0.5 M N,N'-diisopropylcarbodiimide (DIC) in DMF was added to activate C terminus (served as "ACT" step), and the desired amino acid (served as Fmoc-AA(protected side chain)-OH) in a five-fold excess amount (with a concentration of 0.2 M in DMF) and 1.0 M Oxyma in DMF were used for coupling reaction (step AA). The standard coupling reaction was processed (the first stage: a temperature of 75°C, power of 170 W, and a temperature holding time of 15 seconds; the second stage: a temperature of 90°C, power of 30 W, and a temperature holding time of 230 seconds). After conducting AA for about 4 minutes, the Wang resin was washed with DMF solution. The steps of DEP-ACT-AA were repeated to construct a peptide from C-terminus to N-terminus.

Finally, the peptide is removed from the solid support by treating with 95% TFA/2.5% H2O/2.5% TIPS in an ice bath then allowing it to come to room temperature for 2 hours. The filtrate was collected after filtration, added with iced ether for precipitation, and centrifuged to remove supernatant. After that, the ether was added to wash the precipitate. The aforementioned steps were repeated for 3 to 6 times. The final precipitate was taken out and freeze-dried to obtain a white floccus-like product, which is the final product. The purity of the final product was determined by high performance liquid chromatography, and the molecular weight and the sequence were analyzed by LC-MS/MS.

### Preparation Example 2

Arthritis was induced in ICR mice (8 to 12 weeks, 20 to 25 g of body weight, from BioLASCO Taiwan) as follows: the right ankle joint of each mouse was injected with 1 mg/mL Complete Freund's Adjuvant (CFA) continuously for once a week with an amount of 5 µL. The first time of injection is the week 0. After 4 times of injection, long-term inflammation and swollen ankle were induced in the ICR (Institute of Cancer Research) mice on their unilateral front ankle joint for at least 12 weeks, and bilateral mechanical hyperalgesia was also induced continuously for at least 12 weeks, so that served as a model of rheumatoid arthritis suffering long-term chronic pain clinically.

### Preparation Example 3

For conducting nociceptive pain and acid-induced chronic widespread pain experiments, C57BL/6JNarl male mice (20 to 25 g) were housed in an environment with temperature control (24 ± 0.5 °C) and 12-hour light-dark cycle (lights on during 08:00-20:00) and allowed free access to standard laboratory food and tap water. Animals were allowed to acclimatize for 20 to 30 min prior to experimental manipulations. Animal care and handling procedures were adopted in accordance with the International Association for the Study of Pain guidelines for those animals used in pain research, and the procedures were approved by the Committee for Ethics in Animal Research.

### Experiment 1

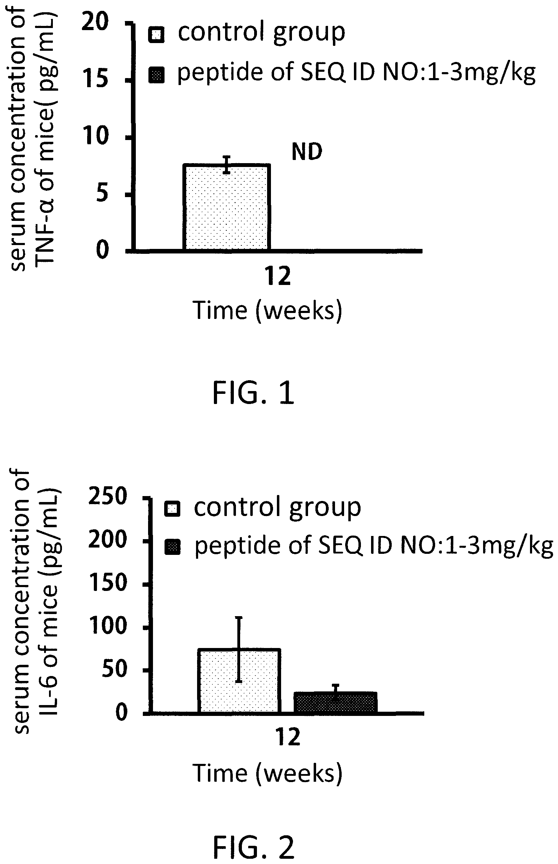

The mice from Preparation Example 2 were orally administered with 3 mg/kg the peptide of SEQ ID NO: 1 dissolved in sterile water on the 4th week, and then continuously administered with the peptide once a week to the 12th week, which were served as the experimental group, comprising 6 mice. On the other hand, the mice administered with water were served as the control group, which also comprised 6 mice. The mice in the experimental group and the control group were sacrificed on the 12th week after administration. The serum collection is conducted by cardiac puncture. The Mouse TNF-α Quantikine ELISA Kit (R&D System, MTA00B), Mouse IL-6 Quantikine ELISA Kit (R&D System, M600B) and enzyme-linked immunosorbent assay (ELISA) were used to determine and collect the serum concentration of TNF-α and IL-6 of mice in the experimental group and the control group. It could be found from the result that the serum concentration of TNF-α of mice in the experimental group, which were subjected to long-term oral administration of the peptide of SEQ ID NO: 1, had decreased to the level out of detectable range, namely not detected (ND) (FIG. 1). Besides, the serum concentration of IL-6 in the experimental group was decreased compared to the control group (FIG. 2). Hence, the peptide of SEQ ID NO: 1 of the present invention can indeed lower the systemic inflammatory indicator, which proves that the peptide of SEQ ID NO: 1 of the present invention can indeed treat the inflammatory disease, especially the chronic systemic inflammation resulting from the rheumatoid arthritis.

### Experiment 2

The mice from Preparation Example 2 were orally administered with 10 mg/kg morphine, an opioid analgesics, on the 4th week, which were served as the positive control. On the other hand, the mice orally administered with 3 mg/kg the peptide of SEQ ID NO: 1 dissolved in sterilized water were served as the experimental group. Both positive control group of morphine and the experimental group comprised 6 mice. Paw withdraw threshold (PWT) of mice was determined at 0 min, 30 min, 60 min, 90 min, 120 min after administration by von Frey Filament.

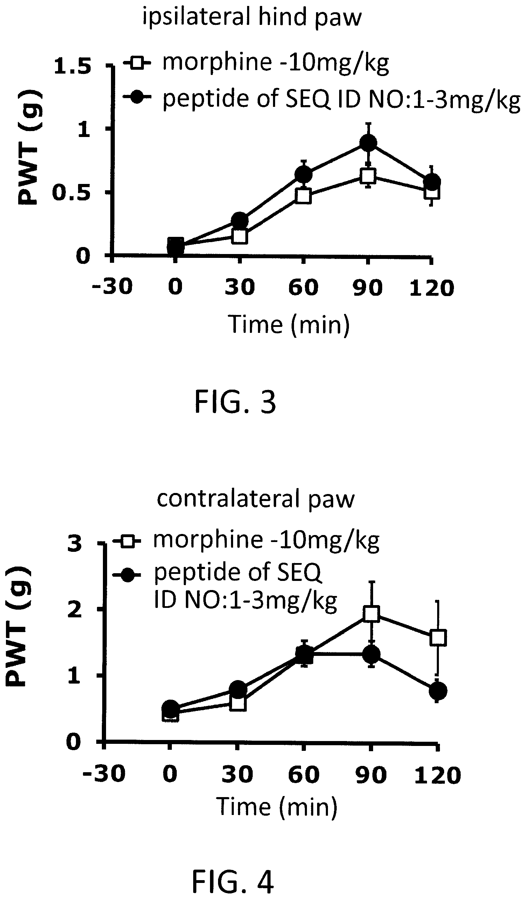

The results of the experiment were shown in FIG. 3 and FIG. 4. After administration with SEQ ID NO. 1, PWT values of ipsilateral hind paw and contralateral paw of the mice in both groups showed an increasing trend as positive control group of morphine. Therefore, this proves that the peptide of SEQ ID NO.1 of the present application has an antinociceptive effect similar to morphine, but a lower amount was required. Besides, it could be observed that the best antinociceptive effect was showed at 90 min after administration in the experimental group and morphine group. At 90 min, in the morphine group, the PWT value of ipsilateral hind paw of mice rose from 0.085±0.015 g to 0.64±0.089 g (as shown in FIG. 3), and the PWT value of contralateral paw of mice rose from 0.43±0.033 g to 1.96±0.488 g (as shown in FIG. 4), while in the experimental group, the PWT value of ipsilateral hind paw of mice rose from 0.07 g to 0.9±0.156 g (as shown in FIG. 3) and the PWT value of contralateral paw of mice rose from 0.5±0.047 g to 1.35±0.193 g (as shown in FIG. 4). Additionally, the antinociceptive effect started to decrease at 120 min after administration..

### Experiment 3

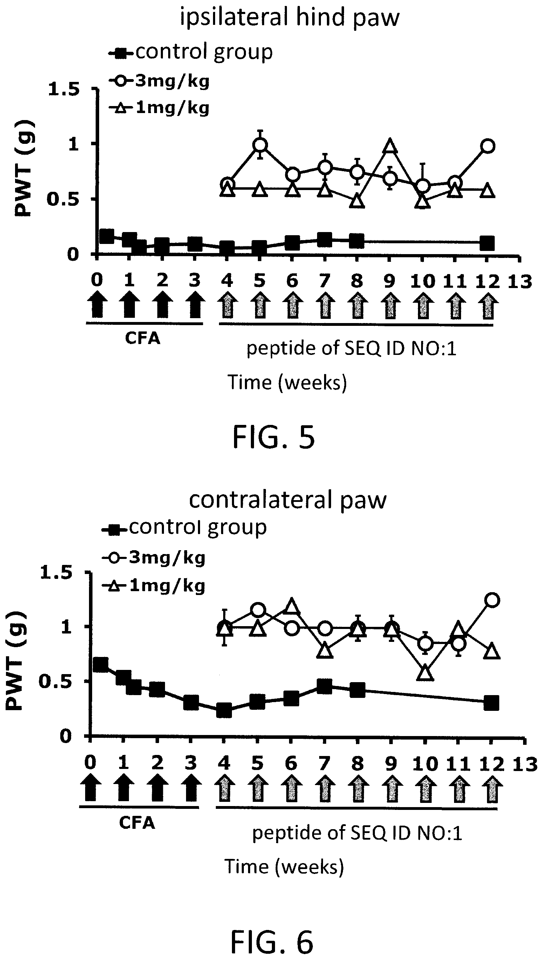

The mice from Preparation Example 2 were orally administered with 1 mg/kg or 3 mg/kg the peptide of SEQ ID NO: 1 dissolved in the sterilized water on the 4^{th} week, and then continuously administered with the peptide once a week to the 12^{th} week, which were shown by gray arrows. The four timings of CFA injection for the mice of the Preparation Example 2 were shown by black arrows in FIG. 5 and FIG. 6. Two dosage groups both contained 6 mice. Mice orally administered with water were used as the control group. The control group also contained 6 mice. The PWT value was measured by the von Frey Filament at 90 min after administration.

The experimental results were shown in FIG. 5 and FIG. 6. After oral administration of 1 mg/kg the peptide of SEQ ID NO: 1, the PWT value of ipsilateral hind paw increased from 0.09 g to 0.5 -1 g (as shown in FIG.5), and the PWT value of contralateral paw increased from 0.4 g at 3^{rd} week to 0.6 - 1.2 g (as shown in FIG. 6). After oral administration of 3 mg/kg the peptide of SEQ ID NO: 1 , the PWT value of ipsilateral hind paw increased from 0.09 g of 3^{rd} week to 0.63-1 g (as shown in FIG.5), and the PWT value of contralateral paw increased from 0.4 g to 0.87-1.27 g (as shown in FIG. 6). Therefore, the results of the experiment showed that the antinociceptive effects of 1 mg/kg and 3 mg/kg of peptide of SEQ ID NO: 1 were similar after long-term administration.

### Experiment 4

24 mice from Preparation Example 2 were provided. The mice were administered with 0.3 µg/kg, 1 µg/kg, or 3 µg/kg peptide of SEQ ID NO: 1 respectively. Every dosage group contained 6 mice. The other 6 mice were orally administered with water, which was served as control group. The PWT value was measured at 90 min after administration by von Frey Filament.

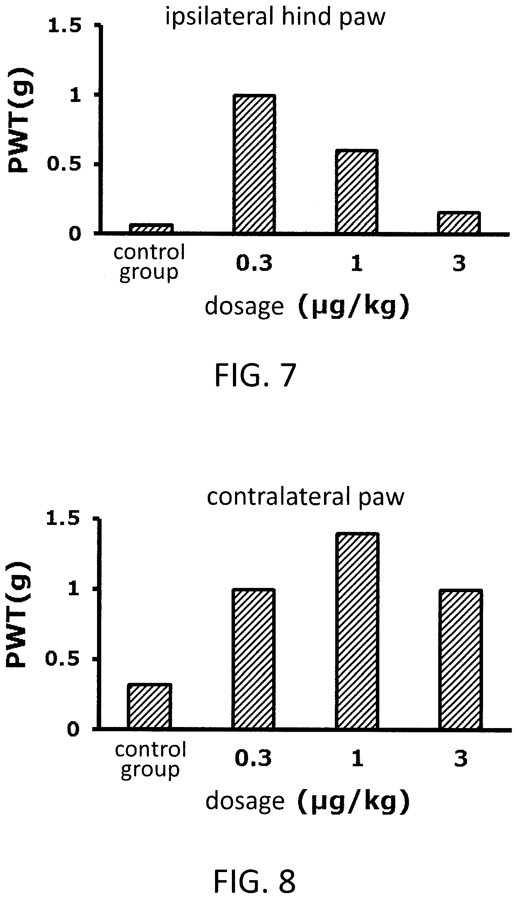

The results were shown in FIG. 7 and FIG. 8. After oral administration of 0.3 µg/kg the peptide of SEQ ID NO: 1, the PWT value of ipsilateral hind paw of mice rose from 0.065 g to 1 g, and the PWT value of contralateral paw rose from 0.32 g to 1 g. After oral administration of 1 µg/kg the peptide of SEQ ID NO: 1, the PWT value of ipsilateral hind paw of mice rose from 0.065 g to 0.6 g, and the PWT value of contralateral paw rose from 0.32 g to 1.4 g. After oral administration of 3 µg/kg the peptide of SEQ ID NO: 1, the PWT value of ipsilateral hind paw of mice rose from 0.065 g to 0.16 g, and the PWT value of contralateral paw rose from 0.32 g to 1 g. The experimental results showed that 0.3 µg/kg to 3 µg/kg peptide of SEQ ID NO: 1 has the antinociceptive effect after oral administration.

### Experiment 5

Acid-induced abdominal constriction assay was performed in accordance with H. O. Collier, L. C. Dinneen, C. A. Johnson, C. Schneider, The abdominal constriction response and its suppression by analgesic drugs in the mouse. Br J Pharmacol Chemother 32, 295-310 (1968). In general, the mice from Preparation Example 3 were placed in a small observation chamber and habituated for 20 min. Distilled water (as a vehicle control) or the peptide of SEQ ID NO: 1 of different concentrations (0.1 µg/kg, 0.3 µg/kg, 1 µg/kg, 0.01 mg/kg, 0.1 mg/kg, 1 mg/kg, 3 mg/kg, and 10 mg/kg) were orally administered to the mice 10 min before the injection of 1% acetic acid (300 µL) which induced a "writhing" response (abdominal constriction) indicative of visceral pain. Vehicle control group and administration groups, which were administered with the peptide of SEQ ID NO: 1, each contained 5 to 12 mice. The numbers of abdominal constrictions in 30 min after acid injection were counted and the mean constriction numbers of mice of each group were calculated. The antinociceptive effect of acid-induced writhing was presented as antinociceptive activity %: (the mean constriction numbers of mice of vehicle control group - the mean constriction numbers of mice of an administration group) / (the mean constriction numbers of mice of vehicle control group) x 100%.

### Experiment 6

The radiant heat tail-flick test was modified in accordance with F. E. D'Amour, D. L. Smith, A method for determining loss of pain sensation. The Journal of Pharmacology and Experimental Therapeutics 72, 74-79 (1941). The 5 to 12 mice from Preparation Example 3 were placed and acclimatized in the restrainers for 30 min prior to the tail-flick test. Heat-flux infrared radiometer (Ugo Basile, Italy, cat. No. 37300) was used for calibrating and maintaining apparatus. A stimulus was applied to the tail of the mouse at the site 2 cm from the tip, and the time taken for the mouse to withdraw its tail from the heat source was defined as the tail-flick latency. To limit the risk of tissue damage, the cut-off time was established at 30.1 seconds (s). Heat intensity was adjusted so that the baseline latencies were set between about 5 and 8 s for most of the animals. The latency was recorded every 10 min during the indicated time period (10-90 min) after oral administration with different dosages of the peptide of SEQ ID NO: 1 (0.01 µg/kg, 0.03 µg/kg, 0.1 µg/kg, 0.3 µg/kg, 1 µg/kg, 0.01 mg/kg, 0.1 mg/kg, 1 mg/kg, 3 mg/kg, and 10 mg/kg). Then the mean latency during 10-90 min was calculated. According to L. S. Harris, A. K. Pierson, Some Narcotic Antagonists in the Benzomorphan Series. J Pharmacol Exp Ther 143, 141-148 (1964), the antinociceptive effect in tail flick test was presented in "%MPE" (maximum possible effect) as defined by the formula: %MPE = (postdrug latency - predrug latency)/(cut-off time - predrug latency) × 100%.

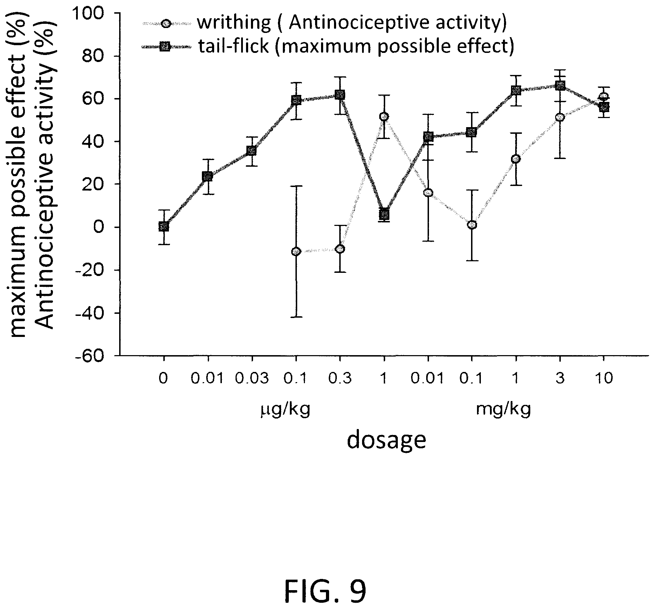

The results of Experiment 5 and Experiment 6 were shown in FIG. 9. The doses of 1 µg/kg and 10 mg/kg the peptide of SEQ ID NO: 1 showed the best antinociceptive effect in the acid-induced writhing experiment, while 0.1 µg/kg, 0.3 µg/kg, 0.1 mg/kg, 1 mg/kg, 3 mg/kg, and 10 mg/kg showed the best antinociceptive effect in the radiant heat tail-flick test. Therefore, the peptide of SEQ ID NO: 1 of the present invention has antinociceptive effect in both high concentration and low concentration.

Consequently, the peptide of the present invention has the effect of treating an inflammatory disease, especially the rheumatoid arthritis, and the effect of treating pain, especially the pain resulting from the rheumatoid arthritis.

The above embodiments are only preferred embodiments of the present invention, not intended to limit the present invention in any aspect. The invention is set out in the appended set of claims.

## Claims

1. A peptide consisting of an amino acid sequence of SEQ ID NO: 1 for use in treating an inflammatory disease.

2. The peptide for use according to claim 1, wherein the inflammatory disease comprises ankylosing spondylitis, osteoarthritis, rheumatic arthritis, rheumatoid arthritis, traumatic arthritis, pyogenic arthritis, gouty arthritis, tuberculous arthritis, neuropathic arthritis, and hemophilic arthritis.

3. A peptide consisting of an amino acid sequence of SEQ ID NO: 1 for use in treating pain.

4. The peptide for use according to claim 3, wherein the pain comprises neuropathic pain, inflammatory pain, musculoskeletal pain, postoperative pain, cancer pain, acute pain, and chronic pain.

5. The peptide for use according to claim 4, wherein the inflammatory pain comprises ankylosing spondylitis pain, osteoarthritis pain, rheumatic arthritis pain, rheumatoid arthritis pain, traumatic arthritis pain, pyogenic arthritis pain, gouty arthritis pain, tuberculous arthritis pain, neuropathic arthritis pain, and hemophilic arthritis pain.

6. The peptide for use according to claim 1 or 3, wherein an effective amount of the peptide ranges from 0.0008 µg/kg to 815 µg/kg.

## Patentansprüche

1. Peptid, enthaltend eine Aminosäuresequenz der SEQ ID NO: 1 zur Verwendung bei der Behandlung einer entzündlichen Erkrankung.

2. Peptid zur Verwendung gemäß Anspruch 1
wobei die entzündliche Erkrankung Spondylitis ankylosans, Osteoarthritis, rheumatische Arthritis, rheumatoide Arthritis, traumatische Arthritis, pyogene Arthritis, Gichtarthritis, tuberkulöse Arthritis, neuropathische Arthritis und hämophile Arthritis umfasst.

3. Peptid, enthaltend eine Aminosäuresequenz der SEQ ID NO: 1 zur Verwendung in der Schmerzbehandlung.

4. Peptid zur Verwendung gemäß Anspruch 3, wobei der Schmerz neuropathischen Schmerz, entzündlichen Schmerz, muskuloskelettalen Schmerz, postoperativen Schmerz, Krebsschmerz, akuten Schmerz und chronischen Schmerz umfasst.

5. Peptid zur Verwendung gemäß Anspruch 4, wobei der entzündliche Schmerz den Schmerz der Spondylitis ankylosans, den Schmerz der Osteoarthritis, den Schmerz der rheumatischen Arthritis, den Schmerz der rheumatoiden Arthritis, den Schmerz der traumatischen Arthritis, den Schmerz der pyogenen Arthritis, den Schmerz der Gichtarthritis, den Schmerz der tuberkulösen Arthritis, den Schmerz der neuropathischen Arthritis und den Schmerz der hämophilen Arthritis umfasst.

6. Peptid zur Verwendung gemäß Anspruch 1 oder 3, wobei eine wirksame Menge des Peptids in dem Bereich von 0,0008 µg/kg bis 815 µg/kg liegt.

## Revendications

1. Peptide constitué d'une séquence d'acides aminés de SEQ ID NO : 1 pour une utilisation dans le traitement d'une maladie inflammatoire.

2. Peptide pour une utilisation selon la revendication 1, dans lequel la maladie inflammatoire comprend la spondylarthrite ankylosante, l'arthrose, l'arthrite rhumatismale, la polyarthrite rhumatoïde, l'arthrite traumatique, l'arthrite pyogène, l'arthrite goutteuse, l'arthrite tuberculeuse, l'arthrite neuropathique et l'arthrite hémophile.

3. Peptide constitué d'une séquence d'acides aminés de SEQ ID NO : 1 pour une utilisation dans le traitement d'une douleur.

4. Peptide pour une utilisation selon la revendication 3, dans lequel la douleur comprend la douleur neuropathique, la douleur inflammatoire, la douleur musculo-squelettique, la douleur postopératoire, la douleur cancéreuse, la douleur aiguë et la douleur chronique.

5. Peptide pour une utilisation selon la revendication 4, dans lequel la douleur inflammatoire comprend la douleur liée à la spondylarthrite ankylosante, la douleur liée à l'arthrose, la douleur liée à l'arthrite rhumatismale, la douleur liée à la polyarthrite rhumatoïde, la douleur liée à l'arthrite traumatique, la douleur liée à l'arthrite pyogène, la douleur liée à l'arthrite goutteuse, la douleur liée à l'arthrite tuberculeuse, la douleur liée à l'arthrite neuropathique et la douleur liée à l'arthrite hémophile.

6. Peptide pour une utilisation selon la revendication 1 ou 3, dans lequel une quantité efficace du peptide est comprise dans la plage allant de 0,0008 µg/kg à 815 µg/kg.
